# EUROPEAN PATENT APPLICATION

(11) **EP 2 856 934 A1**
(43) Date of publication of application: **08.04.2015**
(21) Application number: 13290238.8
(22) Date of filing: 04.10.2013
(51) Int. Cl.: A61B 5/00, A61B 5/055

(54) **Biomarkers for the prediction of long term remission in depression**

(71) Applicant: Les Laboratoires Servier, 92284 Suresnes Cedex (FR)
(72) Inventor: Jabourian, Maritza, 75018 Paris (FR); Fossati, Philippe, 78480 Verneuil SUR Seine (FR); Delaveau, Pauline, 75012 Paris (FR)
(74) Representative: Bestel, Delphine

(57) **Abstract**

Methods for predicting long-term remission among depressed patients. Biomarkers for the prediction of long-term remission in depression.

## Description

The present disclosure relates to methods for predicting long-term remission among depressed patients. In certain embodiments, the invention relates to identify brain regions whose pre-treatment activity could predict the long-term remission of depressed patients with depressant treatment, such as agomelatine.

Two third of patients treated for major depressive disorder (MDD) show a response (symptom reduction >50 %) to an initial antidepressant treatment. However, with a first-line treatment, fewer than 30 % of depressed patients achieved clinical remission, defined as a score of Hamilton Rating Scale for Depression (HAM-D) of 7 or less. Ineffective treatment of depression increases the medical and economic burden of the disorder, puts the patients at risk of suicide and might contribute to the development of treatment-resistant depression and long-term higher relapse rate. Overall this calls for the development of biomarkers that may help the clinician to predict long-term treatment outcome.

Biological predictors such as pre-treatment regional brain activity measured by positron emission tomography (PET) or quantitative activity measured by electroencephalography emerge from brain imaging research. Increased pre-treatment resting rostral anterior cingulate cortex (rACC) activity or reduced ability to suppress rACC activation during cognitive tasks have been proposed as markers of treatment response to antidepressant, electroconvulsive therapy or repetitive transcranial magnetic stimulation in depression. However such predictors have not been adopted in clinical setting due to the relative lack of reliability and validity of these measures. Moreover, up to now, the identified brain imaging markers have been showed to predict clinical response but not clinical remission, which is the primary goal of the treatment.

The main goal of the present study was to use functional magnetic resonance imaging fMRI to identify brain regions whose activity during self-referential processing could predict the long-term remission of MDD patients treated with antidepressant such as agomelatine, an agonist of MT1-MT2 melatonergic receptors and an antagonist of the serotonergic 5-HT2C receptor. To control for learning effect due to the repetition of the self-referential task and assess potential normalization of cerebral activity, we included healthy volunteers receiving placebo for one week. Increased self-focus, the tendency to excessively engage in self-referential processing, is a core feature of acute depression and is associated with abnormal activity of dorsomedial and dorsolateral prefrontal cortex, key regions in the pathophysiology of MDD (7-8). Here we assessed depressed patients making self-referential judgments on emotional pictures during two FMRI acquisitions: 1) before treatment when patients were acutely depressed and 2) following 6 to 7 weeks of treatment with Agomelatine. We hypothesized first that the activity of brain regions associated with self-referential processing at inception would be predictive of clinical remission six months later on.

There is a need in the art for a useful approach to assess a depressed patient, to predict long-term remission in patients with depression and to provide markers for evaluating said prediction of remission.

The present invention provides a method of predicting remission in patients with depression wherein a pre-treatment level of activation in cortical midline regions (CMR) identify patients achieving remission after antidepressant treatment.

"Remission" or "long-term remission" can be defined as a complete or near complete absence of symptoms. There is now a consensus among experts to suggest that achieving long-term remission is the primary goal in the treatment of depression. Patients achieving remission have a lower likelihood of depressive recurrence and a better psychosocial functioning compared to patients with residual depressive symptoms.

By "depression" is meant Major Depressive Episode (MDE) consisting in:
- Major Depressive Disorder (MDD) comprising clinical depression, major depression, unipolar depression, unipolar disorder or as recurrent depression in the case of repeated episodes, is a mental disorder characterized by a pervasive and persistent low mood which is accompanied by low self-esteem and by a loss of interest or pleasure in normally enjoyable activities;
- Bipolar Disorder comprising bipolar affective disorder, manic-depressive disorder or manic depression, is a mental illness classified by psychiatry as a mood disorder. Individuals with bipolar disorder experience episodes of a very high mood known as mania alternating with episodes of depression.

In certain embodiments the present invention relates to method of predicting remission in depressed patients, wherein the antidepressant treatment is selected from the following classes selective serotonin reuptake inhibitors (SSRI), serotonin norepinephrine reuptake inhibitors (SNRI), norepinephrine reuptake inhibitor (NRI), noradrenergic and specific serotonergic antidepressants (NaSSA), norepinephrine-dopamine disinhibitor (NDDI) or tricyclic antidepressant.

Antidepressants are drugs used for the treatment of clinical depression and other conditions.

Antidepressants for which the prediction of remission of the present invention provide results include antidepressants such as citalopram, escitalopram, paroxetine, fluoxetine, duloxetine, milnacipran, venlafaxine, atomoxetine, reboxetine, viloxazine, mianserin, mirtazapine, agomelatine.

In preferred embodiments the present invention relates to method of predicting remission based on activation of CMR at baseline to identify patients achieving remission after agomelatine treatment. Agomelatine or N-[2-(7-methoxy-1-naphthyl)ethyl]acetamide, has the double characteristic of being, on the one hand, an agonist of receptors of the melatoninergic system and, on the other hand, an antagonist of the 5-HT2C receptor.

The invention provides method of remission prediction, wherein the activated cortical midline regions are dorsomedial prefrontal cortex (dmPFC) and precuneus during self-referential processing. The present invention identifies brain regions, specifically dorsomedial prefrontal cortex and precuneus in cortical midline regions, whose activity during self-referential processing predict long-term remission among patients in depression treated with antidepressants.

The precuneus or the quadrate lobule of Foville or Brodmann Area 23 (BA23) is a part of the superior parietal lobule forward of the occipital lobe. The dorsomedial prefrontal cortex or dmPFC or DMPFC, preferably dorsomedial prefrontal cortex BA10 (dmPFC10), has been shown to be involved in attending different states, all including a strong emotional component.

In some embodiments the invention provides method of predicting remission based on activation in cortical midline regions at baseline discriminating patients achieving remission after antidepressant treatment, wherein the patients are suffering from Major Depressive Episode (MDE) preferably Major Depressive Disorder (MDD) or Bipolar Disorders.

Pretreatment activation in the dmPFC (BA10, BA8) and the precuneus during self-referential processing distinguished future remitters from non-remitters. Activation in these three regions was lower in remitters than in non-remitters and remained stable after 6/7 weeks of treatment in both groups of patients. Moreover pretreatment activation of dmPFC10 and precuneus predicted clinical remission at 6 months with a perfect sensitivity (100%) and a good specificity (71.6%).

Therefore, the level of activation of CMR during self-referential processing serves as a marker for predicting remission in a patient as described in the present invention. The present invention relates to methods of remission prediction wherein the activation of cortical midline regions during self-referential processing is lower in remitter patients in comparison with non-remitter patients. The invention encompasses a method of prediction remission in depressed patient wherein the cut-off level of remitter patients is determined by the discrimination ability of mean blood oxygen level dependent (BOLD) signal in function of the receiver operating characteristic (ROC) curve.

Actually, we used receiver operating characteristic (ROC) curve analysis to assess the discrimination ability of mean BOLD signal region of interest (ROIs) activity resulting from the contrast estimate between self-referential and general condition processing at baseline (W0), using SPSS 15.0 software. Then, from the ROC curve, we determined a cut-off value that maximized percentage correct discrimination, from the Youden index, equal to the sensitivity + specificity - 1. As an example, the optimal cut-off from the dmPFC10 activation (i.e. 7.1) led to 81.25% correct prediction of remission and from the precuneus activation (i.e. 6.5) led to 87.5% correct prediction (for both, sensitivity of 100% and specificity of 71.6%).

Precuneus and dmPFC10 activation during self-referential processing at baseline predicted clinical changes at 24 weeks. Using baseline activation of these two regions, we were able to predict with robust specificity and sensitivity the long-term clinical outcome of patients. Pattern of brain activity clearly differentiated remitters from non-remitters suggesting cross-scanner generalizability and validating the clinical utility of such assessment. Consistent with previous studies, our results emphasized the role of cortical midline structures or regions (CMS) (including the dmPFC and precuneus) in self-referential processing and depression. Increased self-focus and impaired functioning of the CMS are crucial in the development and course of MDD. The present study is consistent with the idea that cortical midline structures activity play a major role in treatment outcome of MDD and could inform the choice of this treatment.

The present invention provides method of prediction remission whereby the pre-treatment activation or activation at baseline is measured by functional magnetic resonance imagery (fMRI).

Functional magnetic resonance imaging or functional MRI (FMRI) is a functional neuroimaging procedure using MRI technology that measures brain activity by detecting associated changes in blood flow. This technique relies on the fact that cerebral blood flow and neuronal activation are coupled. When an area of the brain is in use, blood flow to that region also increases. The primary form of FMRI uses the Blood-oxygen-level dependent (BOLD) contrast.

In other embodiment the prediction of remission is validated if the long-term remission is achieved after 24 months, 12 months or preferably 6 months antidepressant treatment.

The present invention concerns agomelatine for use in treating a patient in need thereof, wherein the patient is identified by the method of prediction long-term remission according to the invention.

In other embodiments the invention relates to the use of pre-treatment activation in CMR for predicting long-term remission in patients with depression after depressant treatment.

The present invention is illustrated by the following Figures and Examples, without being limited thereby :
Figure 1 : Study plan design
Figure 2 : A. Example of fMRI experimental design. B. Example of stimuli presentation. For each picture, subjects gave either a 'yes' or 'no' response for the self condition, a 'positive' or 'negative' response for the general condition or a 'indoor' or 'outdoor' response for the 'control' condition by pushing a button with the right- or the left- hand thumb.
Figure 3 : Demographic and clinical characteristics of MDD patients and Healthy Volunteers at baseline
Figure 4 : Demographic and clinical characteristics at baseline of future remitted and non-remitted patients 6 months later on.
Figure 5 : A. Mean BOLD signal from the regions of interest (main effect of group, ANOVA 2) in future remitters and non-remitters (at W24), at W0 and W7: dmPFC10 (MNI coordinates, -4 56 12), dmPFC8 (-10 48 42), precuneus (-8 -62 20). The bars graphs plot the contrast estimates between self-referential and general conditions per group and scan time. B. Negative correlation between the percentage of decrease in HAM-D score between the time W0 and W24 and the mean BOLD signal from dmPFC10, dmPFC8 and precuneus in self versus general condition at baseline (W0).
Figure 6 : Receiver operating characteristic (ROC) curves for remission computed using mean signal regions of interest (main effect of group, ANOVA 2) at baseline (W0).

### Example : Identification of biomarkers to predict remission in depression

### Subjects

Twenty-five female outpatients meeting the Diagnostic and Statistical Manual of Mental Disorders-IV (DSM-IV, American Psychiatric Association) criteria for MDD with the Mini International Neuropsychiatric Interview were recruited between October 2008 and June 2011 by psychiatrists (seven centres located in Paris area, France). Current depressive episode had to be of moderate or severe intensity: 17-item Hamilton Depression Rating Scale (HAM-D; 10) total score ≥22 and Clinical Global Impression (CGI; 11) severity score ≥4. Patients had to be free of psychotropic medication for a minimum of two weeks (washout period defined according to the medication) before inclusion. Patients with comorbid psychiatric conditions were excluded.

Before each fMRI scanning, a drug screening (for cannabinoids, opiates, amphetamines, metamphetamines, cocaine, and benzodiazepines), and alcohol breath were performed and subjects were excluded from analyses if positive. Four patients were excluded for positive screening for benzodiazepines before performing the third fMRI scan and two patients did not perform the third scan leading to a final sample of 19 MDD patients. They were compared to 14 healthy control subjects. Healthy volunteers were in good health as assessed by a complete medical questionnaire including medical history, a physical examination, and a psychiatric interview. They were not selected if there was any previous or ongoing chronic or recurrent disease of the central nervous system or psychiatric disorder (documented by the Mini International Neuropsychiatric Interview) or a family history of bipolar disorder or recurrent depression. None of the healthy volunteers used drugs acting on central nervous system.

### Study design and drug treatment

Each patient was scanned on three occasions, before beginning the treatment (W0), after one week (W1) and seven weeks of treatment (W7). The controls were scanned on two occasions, at W0 and after one week (W1).

The study comprised two periods of treatment, one double-blind, placebo-controlled period of one week, with two parallel groups of MDD patients having taken either 1 tablet of agomelatine 25 mg (AGO) or one tablet of placebo (PBO), orally per day at around 8 p.m, during seven days in double-blind condition), followed by a period with agomelatine 25 mg until 24 weeks (W24).

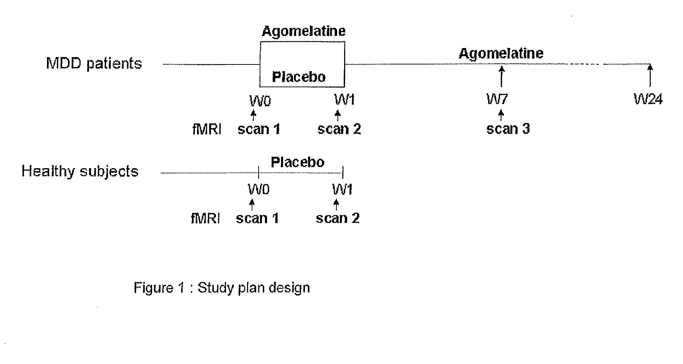

All controls took one tablet of placebo orally per day at around 8 p.m., during seven days, in single-blind condition. (Figure 1)

### Clinical assessment

Depression severity was assessed by the 17-item HAM-D scale and CGI severity and clinical improvement sub-scores before each FMRI scan (W0, W7) and after 24 weeks of treatments (W24) in MDD patients.

### Experimental design

A total of 432 black and white pictures were taken from either the International Affective Picture System (IAPS) or the Empathy Picture System (EPS). 36 positive images, 36 negative images and 36 neutral images were used during each fMRI scanning and 36 used during practice sessions before fMRI scanning (12 per valence). So, each picture was seen only once by subjects during the fMRI scans.

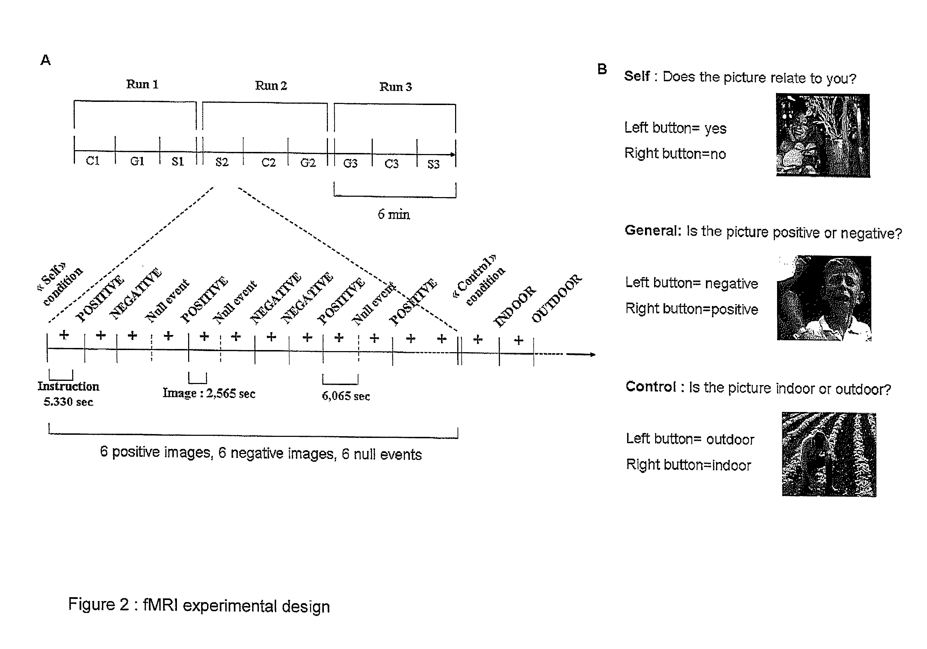

The task comprised three judgment conditions: self, general and a control condition. In both self and general conditions, the subjects were presented with an equal number of positive and negative pictures. In the control condition, subjects were presented with neutral pictures. In the self condition, the subject judged if the picture was self-related or not. In the general condition, the subject judged if the picture was positive or negative. In the control condition, the subject had to state if the picture represented an outdoor or indoor scene (Figure 2).

### Clinical and behavioural data analysis

The analysis of clinical scales and task performance was conducted using the Statsoft Statistica version 10 (www.statsoft.fr) statistical package. Differences in demographic data (age, education) between both groups (patients and healthy subjects) were analysed with a two-sample Student t-test. Differences of ratings in HAM-D-17 scores between W0 and W7 (19 MDD patients), and between W7 and W24 (16 MDD patients) were compared using a paired-t test.

Differences in clinical characteristics between the two sub-groups of MDD patients (remitted and non-remitted at 24 weeks) before any treatment were compared using the parametric Student t-test or, when the population was not normally distributed (defined by the Kolmogorov-Smirnov test), the non-parametric test Mann-Whitney U-test for age, duration of disease, of current episode, number of previous depressive episodes and HAM-D-17 score, and using the chi-square test for DSM-IV classification.

Difference in task performance (accuracy score and reaction time during self, general and control conditions) were compared using an analysis of variance with a between-group factor (MDD patients, healthy subjects), and 3 within-group factors: condition factor (self/general/control), valence factor (positive/negative), scan time factor (W0/W7 for patients and W0/W1 for controls).

The threshold for statistical significance was fixed at p<0.05 (2-tailed).

### fMRI data preprocessing

Data were processed using the SPM5 software (http://www.fil.ion.ucl.ac.uk/spm/software/spm5). A standard data preprocessing was performed: slice timing, movement correction, co-registration with the T1-weighted image and normalization into a standardized MNI (Montreal Neurological Institute) coordinate system space using the transformations computed during the segmentation of the T1-weighted image, and finally spatial smoothing with an isotropic kernel of 8 mm full-width at half-maximum. An individual statistical parametric map was computed for each subject using the general linear model. Each condition was modelled using an event-related approach and convolved with the canonical hemodynamic response function (HRF) to create regressors of interest (self, general and control). A high-pass filter (cut-off of 128 seconds) was applied and motion realignment parameters included as regressors of non-interest. The following fist-level individual t-contrast images were obtained for the HRF estimates: "self versus general", "self versus control" and "general versus control" condition.

Because we did not observe any significant interaction between condition, valence and group at the first and second sessions (p<0.005) we did not take into account emotional valence.

### Associations between pre-treatment brain activation at W0 and clinical change at W24

First we used HAM-D (i.e. the independent variable) as a categorical variable, to split the MDD sample in two groups according to a HAM-D score cut-off of 7 after 24 weeks of treatment (remitters ≤7, non-remitters >7). Subsequently, we performed an ANOVA on "self versus general" contrast images (i.e. the dependent variable) with a between-group factor (remitters versus non-remitters) and a within-group scan time factor (W0 versus W7) (significant threshold p<0.001 uncorrected, size of cluster≥10 voxels).

Second, we focused the analysis on the brain regions functionally defined on the basis of this ANOVA. The resulting peak coordinates were used as centers of 5-mm radius spheres and the mean signal was extracted from the ROIs at W0 using Marsbar software (http://marsbar.sourceforge.net). We used percentage of decrease in HAMD score between the time W0 and W24 as a continuous variable, computing Pearson's correlation coefficients with the mean signal ROIs (using the Statsoft Statistica version 10 statistical package).

Finally, to predict remission at 24 weeks, we used receiver operating characteristic (ROC) curve analysis to assess the discrimination ability of mean signal ROIs activity during self-referential processing at baseline (W0), using SPSS 15.0 software. Then, from the ROC curve, we determined a cut-off value that maximized percentage correct discrimination, from the Youden index, equal to the sensitivity + specificity - 1 (15).

### Demographic data

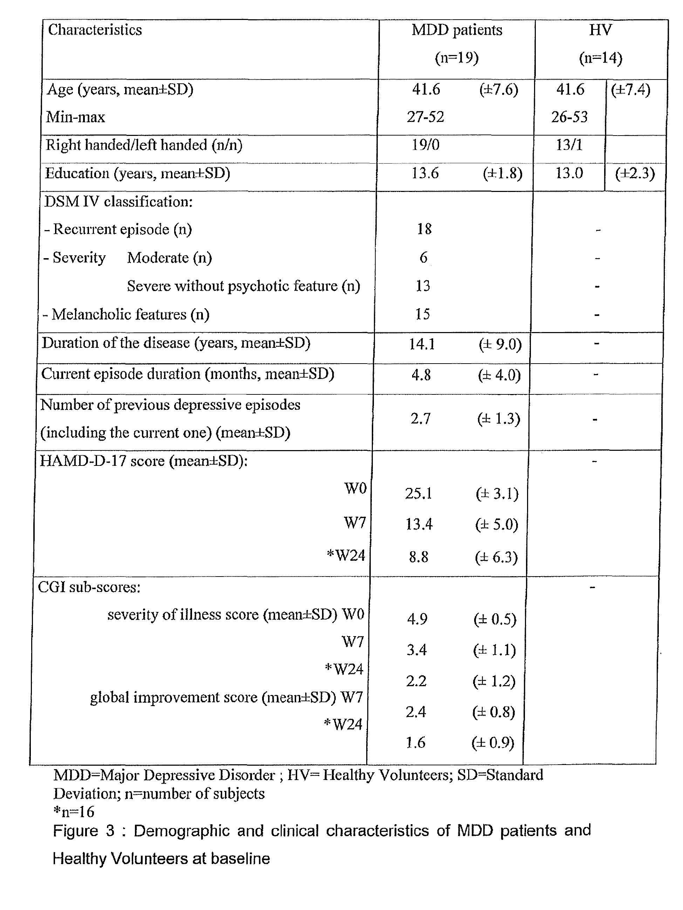

Figure 3 summarizes demographic and clinical characteristics of patients and controls. No significant difference was found between the two groups for age or education level.

### Clinical effects

No clinical difference in HAM-D scores was found before treatment between patients receiving Agomelatine for 6 or 7 weeks (PBO/AGO, n=10 patients, HAM-D=24.9±2.8; AGO/AGO, n=9 patients, HAM-D= 25.2±3.6; p=0.4).

The mean score of HAM-D decreased from W0 to W7 (25.1±3.1 versus 13.4±5.0; t(18)=11.8, p<0.001) and from W7 to W24 (8.8±6.3; t(15)=2.8, p=0.014; n=16 patients). At W7, ten patients (62,5%) were responders (score reduction ≥50%), seven patients were partially responders (score reduction between 30 and 50%) and two patients were non-responders (score reduction < 30%).

Regarding CGI scores, the severity of illness at W0 decreased from "markedly" (mean=4.9±0.5) to "mildly"/"moderately" (3.4±1.1) at W7 and to "borderline mentally" (2.2±1.2) at W24. Patients were "minimally" to "much" improved at W7 (mean=2.4±0.8) and "much" to "very much" improved at W24(1.6±0.9) (Figure 3).

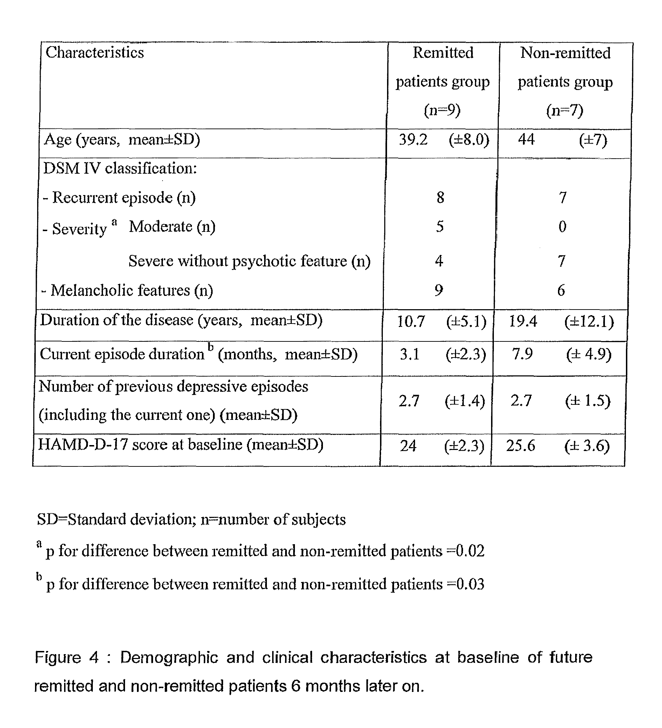

Among the 16 patients included in the analyses of prediction, after 24 weeks of treatment we were able to distinguish nine remitters (56.2%) from seven non-remitters. At W0, future remitters and non-remitters did not differ for age, duration of disease, number of depressive episodes and severity of illness according to HAM-D score. The duration of the current episode was longer in non-remitters patients (7.9±4.9 months) as compared to remitters (3.1±2.3 months; U=11, p=0.03). According to the DSM-IV, there were more severe major depressive episodes in non-remitters compared to remitters patients (χ²=5.7, p=0.02) (Figure 4).

### fMRI results : Associations between pre-treatment brain activation and clinical change at 24 weeks

Comparing remitters (n=9) and non-remitters (n=7), we observed a significant main effect of group in the dorsomedial prefrontal cortex BA10 (dmPFC10) (MNI coordinates: -4, 56, 12; F[1,28]=18.4; small volume correction [SVC] 5mm-radius, t[28]=4.29, p=0.002, 31 voxels), the dorsomedial prefrontal cortex BA8 (dmPFC8) (MNI coordinates: -10, 48, 42; F[1,28]=24.6; SVC 5mm-radius, t[28]=4.96, p=0.001, 49 voxels) and the precuneus BA 23 (MNI coordinates: -8, -62, 20; F[1,28]=19.6; SVC 5mm-radius, t[28]=4.42, p=0.002, 31 voxels). There was no effect of scan time or group × scan time interaction suggesting that activation in these brain regions remained stable between W0 and W7.

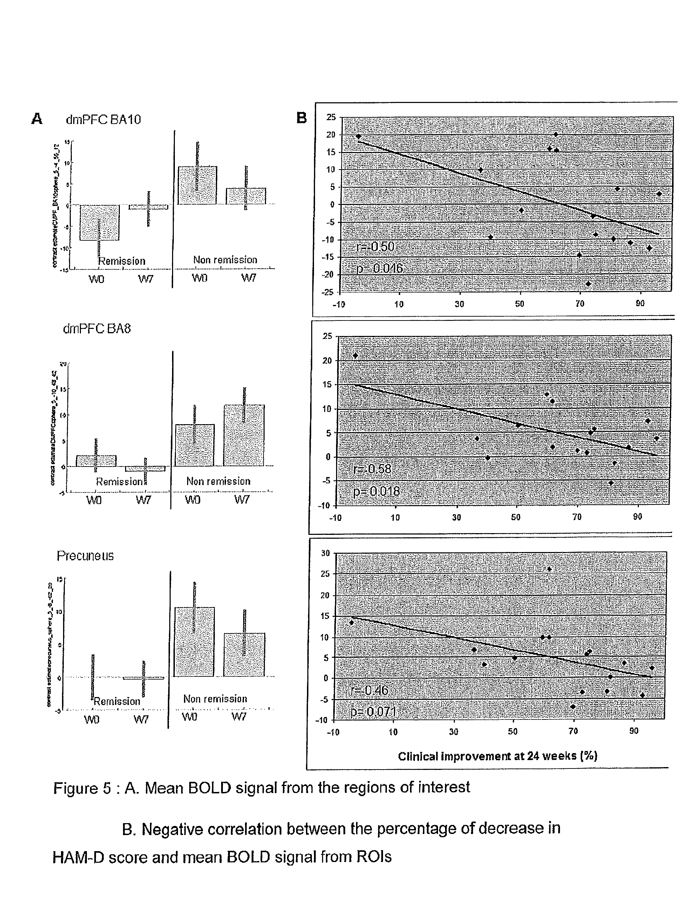

The main effect of group was explained by a reduced activation in these three regions at baseline in future remitters compared to future non-remitters at both W0 and W7 (Figure 5A).

We tested the relationship of baseline activation in the dmPFC8, dmPFC10 and precuneus with clinical changes. There was a significant negative correlation between the percentage of decrease in HAM-D score from W0 to W24 and the activation at W0 in the dmPFC10 (r=-0.50, t(14)=-2.19, p=0.046), the dmPFC8 (r=-0.58, t(14)=-2.69, p=0.018) and a trend toward a significant negative correlation with the precuneus activation (r=-0.46, t(14)=-1.95, p=0.071). Consistent with the categorical analysis comparing remitters and non-remitters, the less these three cortical midline regions were activated at baseline during self-referential processing, the better the improvement of clinical symptoms after 24 weeks of treatment by Agomelatine (Figure 5B).

### ROC (Receiver Operating Characteristic) analyses and prediction of remission at 24 weeks

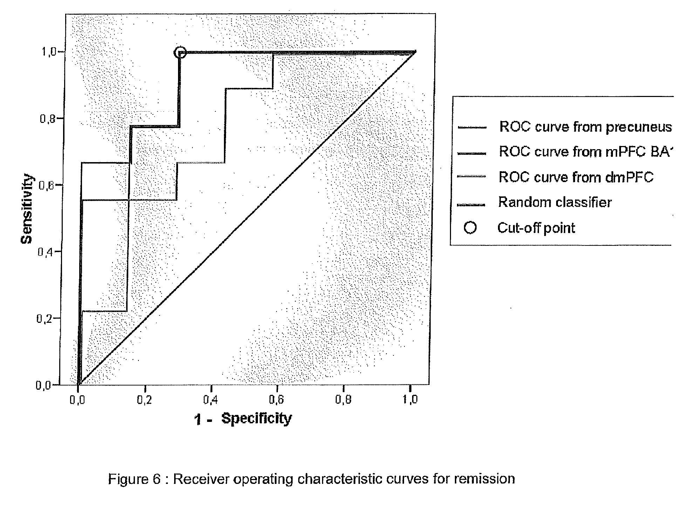

We took advantage of the individual variability of long-term clinical outcome in order to evaluate the accuracy of brain activation in dmPFC10, dmPFC8 and precuneus as predictors of remission at 24 weeks. The ROC curves yielded a significant area under the curve (AUC) for the dmPFC10 (AUC=0.905, p=0.007), the precuneus (AUC=0.921, p=0.005), and a tendency for the dmPFC8 (AUC=0.762, p=0.081) reflecting a good discrimination between future remitters and non-remitters from the activation of dmPFC10 and precuneus at baseline. The optimal cut-off led to 81.25% correct prediction of remission from the dmPFC10 activation and 87.5% correct prediction from the precuneus activation (for both, sensitivity of 100% and specificity of 71.6%) (Figure 6). Remitters were characterized by lower pre-treatment activation of dmPFC10 and precuneus during self-referential processing whereas higher activation of dmPFC10 and precuneus was associated with poor prognosis at 24 weeks.

## Claims

1. Method of predicting remission in patients with depression wherein a level of activation at baseline in cortical midline regions (CMR) identify patients achieving remission after antidepressant treatment.

2. Method of claim 1, wherein the antidepressant treatment is selected from the antidepressant classes consisting in : selective serotonin reuptake inhibitors (SSRI), serotonin norepinephrine reuptake inhibitors (SNRI), norepinephrine reuptake inhibitor (NRI), noradrenergic and specific serotonergic antidepressants (NaSSA), norepinephrine-dopamine disinhibitor (NDDI) or tricyclic antidepressant.

3. Method of claim 2, wherein the antidepressant is selected from the group consisting in : citalopram, escitalopram, paroxetine, fluoxetine, duloxetine, milnacipran, venlafaxine, atomoxetine, reboxetine, viloxazine, mianserin, mirtazapine, agomelatine.

4. Method of claim 3, wherein the antidepressant is agomelatine.

5. Method of claim 1, wherein the cortical midline regions are dorsomedial prefrontal cortex (dmPFC) and precuneus.

6. Method of claim 1, wherein the long-term remission is predicted in patients suffering from Major Depressive Episode (MDE) comprising Major Depressive Disorder (MDD) and Bipolar Disorders.

7. Method of claim 1, wherein the activation of cortical midline regions during self-referential processing is lower in remitter patients in comparison with non-remitter patients.

8. Method of claim 1, wherein the patients achieving remission after antidepressant treatment are under the cut-off level determined by the discrimination ability of mean blood oxygen level dependent (BOLD) signal in function of the receiver operating characteristic (ROC) curve

9. Method of claim 1, wherein the pre-treatment activation is measured by functional magnetic resonance imagery (fMRI).

10. Method of claim 1, wherein the remission is achieve after 6 months antidepressant treatment.

11. agomelatine for use in treating a patient in need thereof, wherein the patient is identified by the method of prediction long-term remission of claims 1 to 10.

12. Use of pre-treatment activation in CMR for predicting long-term remission in patients with depression after depressant treatment.
